# EUROPEAN PATENT SPECIFICATION

(11) **EP 3 081 207 B1**
(45) Date of publication and mention of the grant of the patent: **21.09.2022**
(21) Application number: 15163940.8
(22) Date of filing: 16.04.2015
(51) Int. Cl.: A61K 8/34, A61K 8/49, A61Q 5/00, A61Q 19/02, A61K 8/14

(54) **USE OF A LIPOSOME COMPOSITION**
VERWENDUNG EINER LIPOSOMENZUSAMMENSETZUNG
UTILISATION D'UNE COMPOSITION DE LIPOSOMES

(43) Date of publication of application: 19.10.2016
(73) Proprietor: Symrise AG, 37603 Holzminden (DE)
(72) Inventor: Vielhaber, Gabriele, 92700 Colombes (FR); Meyer, Imke, 37619 Bodenwerder (DE); Lange, Sabine, 37603 Holzminden (DE)
(74) Representative: Fabry, Bernd

(56) References cited:
- WO-A2-2009/087242
- WO-A2-2009/105294
- JP-A- 2013 053 104
- JP-A- 2013 071 890
- US-B1- 6 280 754
- DATABASE GNPD [Online] MINTEL; August 2012 (2012-08), "Smoth sailing Anti-Frizz", XP002745394, Database accession no. 1824858
- DATABASE GNPD [Online] MINTEL; May 2012 (2012-05), "Ultra Super White 377", XP002745395, Database accession no. 1768209
- DATABASE GNPD [Online] MINTEL; July 2008 (2008-07), "Maxi White Anti Dark Spot serum", XP002745396, Database accession no. 947069
- DATABASE GNPD [Online] MINTEL; July 2008 (2008-07), "Ultimate Whitening Bio-Cellulose mask", XP002745397, Database accession no. 949873
- MICHI GOHARA, AKIKO YAGAMI, KAYOKO SUZUKI ET AL: "Allergic contact dermatitis caused by phenylethyl resorcinol [4-(1-phenylethyl)-1,3-benzenediol], a skin-lightening agent in cosmetics", CONTACT DERMATITIS, vol. 69, no. 5, 12 October 2013 (2013-10-12), pages 319-320, XP002745398, DOI: 10.1111/cod.12114

## Description

### FIELD OF INVENTION

The present invention belongs to the area of whitening or brightening compositions, which are in particular formulated into cosmetic preparations and which are especially for the application to skin or keratinous surfaces, such as scalp.

### TECHNOLOGICAL BACKGROUND

The whiteness and transparency of the skin have traditionally been basic requirements for polished beauty in Asia. In the last years, a lot of cosmetic skin-whitening preparations have been developed. Some of them directly act on the biosynthetic mechanism of melanin, e.g. through the use of compounds such as kojic acid, L-ascorbic acid and derivative thereof, various sulfur compounds, arbutin and the like, which inhibit the activity of an oxidase, tyrosinase which promotes a melanin synthesis or even inhibit the synthesis of tyrosinase in first line.

In the past years, the interest for skin whitening (or brightening), however, also arises in the western countries. The main interest in skin whitening is besides beauty and affluence, the treatment of skin issues such as uneven pigmentation that arises from excessive sun exposures, age spots, freckles, and so on.

In any event, a lot of skin whitening compounds show excellent efficacy in whitening or brightening skin, but can be irritating or stinging to sensitive skin. In other cases the skin whitening compounds show good capability but have less optimal efficacy, because the formula in which the whitening compound is present, leads not to an optimal absorption into the skin.

### RELEVANT STATE OF THE ART

WO 2009087242 A2 (SYMRISE) relates to the use of trans-tert-butyl cyclohexanol as skin irritation-reducing agent as well as compositions (formulations) having a skin irritation-reducing action comprising trans-tert-butyl cyclohexanol as skin irritation-reducing agent.

US 6,280,754 B1 (TAGASAGO) discloses a dermal topical formulation containing p-menthane-3,8-diol and a whitening component such as arbutin, kojic acid, ellagic acid, ascorbic acid or its derivative, lactic acid, glycolic acid, tartaric acid, and an essence or an absolute extracted from a plant such as labdanum, jasmine and mugwort (T.vulgare).

JP 2013 071890 A (IWASE) refers to a cosmetic composition including 2-ethylhexanoic acid 2-(2-ethyl-hexanoyloxy)-cyclohexyl ester; and one or more kinds of bleaching components selected from a group consisting of a hydroquinone glycoside or its derivative, L-ascorbic acid or its derivative, resorcinol or its derivative, tranexamic acid or its derivative, kojic acid and a placental extract.

JP 2013 053104 A (TAKARA BELMONT) discloses a skin irritation relieving agent for cosmetics containing caffeine and 4-t-butylcyclohexanol which is mixed and combined with the cosmetic composition, or used prior to use of the cosmetic composition.

### OBJECT OF THE INVENTION

Accordingly, an object of this invention was to provide a stable preparation comprising a whitening agent, which is preferably a skin whitening agent, but which can also be applied to keratinous surfaces like the scalp. In particular, an object of the invention was to provide a composition with the whitening agent when formulated into cosmetic or pharmaceutical preparations shows reduce /decrease or less stinging, itching and pricking of the skin. A further object was, in particular, to formulate a stable preparation with the whitening agent, especially in preparations such as skin whitening preparations, leave-on hair products and hair colorations, and simultaneously keep the whitening capability optimal efficacy. Further object was to improve the feeling of the skin through the reduction / decrease of stinging, itching and pricking caused by the whitening agent.

### DESCRIPTION OF THE INVENTION

Subject of the present invention is the use of a liposome composition comprising
(a) at least a whiting or brightening agent, selected from the group consisting of diphenylmethanes, sclareolide; Tetraselmis Suecica Extract; licorice extract; pomegranate extract; hinokitiol; protocatechuic acid; NAB asafetida (Ferula Foetida) extract; resveratrol oxyresveratrol resveratrol phosphate, resveratrol ferulate; ferulic acid ferulic acid phosphate; viniferol; mixtures of Saxifrage, Grape, mulberry and Scutelleria Root extracts, mixture of cucumber, apple and Scutellaria extracts or mixtures of cucumber, apple and Scutellaria, and green tea extracts; mixtures of butylene glycol/water/Denothera Biennis seed extract; evening primrose extract; fatty acid esters of ascorbic acid, ascorbyl palmitate; Euphrasia Officianalis extract, kinetin; ascorbyl glucoside; grape seed extract; tetrahydrocurcumins, Acmella Oleracea extract, Aloesin, extracts of field dock, aspergillus ferment, molasses, 4-(1-Phenylethyl) 1-,3 benzenediol, and
(b) at least one solubilizer selected from the group consisting of Tetratglyceryl Monocaprate (Polyglyceryl-4-Caprate), Triglyceryl Monocaprate (Polyglyceryl-3-Caprate), Polyglyceryl-2 Sesquiisostearate, Decyl-Glucoside Sorbitan Oleate Crosspolymer, Lauryl-Glucoside Sorbitan Oleate Crosspolymer,
   and optionally
(c) at least one physiological cooling agent
   for the manufacturing of a cosmetic preparation selected from the group consisting of skin whitening or brightening preparations, leave-on hair products and hair coloration products.

Surprisingly, it has been observed that the use of the composition as defined above leads to less irritation and/or stinging and/or picking, especially to the skin.

### Whitening and brightening agents

Preferably the whitening agent is 4-(1-Phenylethyl) 1-,3 benzenediol.

In another preferred embodiment the composition of the present invention comprises the whitening or brightening agent, which is preferably a skin whitening agent or hair brightening agent, in combination with cis- and/or *trans-* 4-tert-Butylcyclohexanol and/or 1, 3, 7-Trimethyl-3,7-dihydro-1H-purin-2,6-dion (Caffeine) or derivatives thereof.

Especially the use of brightening or whitening agents in cosmetic products, in particular cosmetic products for hair coloration, often induce burning and stinging of the scalp. The component cis- and/or *trans-* 4-tert-Butylcyclohexanol when formulated in such preparations have an impact on this disadvantage and is able to especially reduce/decrease the burning and stinging of the scalp.

The combination of the whitening or brightening agent with cis- and/or *trans-* 4-tert-Butylcyclohexanol also shows the advantage of reduced and decreased stinging, itching and pricking of the skin. Preferably a mixture of cis- and *trans-* 4-tert-Butylcyclohexanol is used, but also only cis- or *trans-* 4-tert-Butylcyclohexanol may also be used. More preferably, cis- and/or *trans-* 4-tert-Butylcyclohexanol solubilized in pentylene glycole is used, which is available under the trade name SymSitive^{®}1609 by Symrise AG.

The combination of the whitening or brightening agent with 1, 3, 7-Trimethyl-3,7-dihydro-1H-purin-2,6-dion (Caffeine) or derivatives thereof has shown that the stinging, itching and pricking of the skin could be decreased through the impact of the caffeine, respectively the derivative thereof. Preferably the whitening agent is 4-(1-Phenylethyl) 1-,3 benzenediol (SymWhite 377 ^{®}).

In a preferred embodiment the liposome compositions may further comprise
(d) ethanol,
(e) 1,3,7-Trimethyl-3,7-dihydro-1H-purin-2,6-dion (Caffeine),
(f) cis- and/or trans- 4-tert-Butylcyclohexanol,
   or a mixture thereof.

Suggested ranges of whitening agents in a composition (which is not the end product) are from 0.001 to 95% b.w., preferably from 0.005 to 90% b.w., more preferably from about 0.01 to 85% b.w. of the total composition.

### Solubilizers

The at least one solubilizer is preferably a non-ethoxylated solubilizer, preferably not a PEG compound. For that it is known that PEGs supporting the penetration of skin, it is desirable to find alternatives to PEGs, respectively to find substances may be used in combination with PEGs to reduce the amount of PEGs in cosmetic products. The solubilizer are therefore selected from Tetraglyceryl Monocaprate (INCI: Polyglyceryl-4-Caprate), Triglyceryl Monocaprate (INCI: Polyglyceryl-3-Caprate), Polyglyceryl-2 Sesquiisostearate, Decyl-Glucoside Sorbitan Oleate Crosspolymer, Lauryl-Glucoside Sorbitan Oleate Crosspolymer or a mixture thereof.

The advantage in using the solubilizer as mentioned is that on the one hand the solubilizer is not ethoxylated (PEG free), thus the disadvantages of PEG containing composition do not exist here, and on the other hand that the whitening agent is pre-solubilized, thus when formulated into cosmetic compositions the stinging, itching and pricking caused by the whitening agent could be reduce/ decrease.

### Physiological cooling agents

The at least one cooling agent is preferably selected from the group consisting of Menthyl Lactate (Frescolat ML), Menthone Glycerin Acetal (Frescolat MGA), Menthyl Ethylamido Oxalate (Frescolat X-Cool), menthol and menthol derivatives (e.g. L-menthol, D-menthol, racemic menthol, isomenthol, neoisomenthol, neomenthol), 5-methyl-2-(propane-2-yl)cyclohexyl-N-ethyloxamate, N-ethyl-p-menthane carboxamide (WS-3, also referred to as menthane-3-carboxylic acid-N-ethyl amide), N-2,3-trimethyl-2-isopropyl butane amide (WS-23), menthyl lactate (Frescolat.RTM. ML), menthone glycerine acetal (Frescolat.RTM. MGA), mono-menthyl succinate (Physcool.RTM.), mono-menthyl glutarate, O-menthyl-glycerine, menthyl-N,N-dimethyl succinamate, N-(4-cyano methyl phenyl)-p-menthane carboxamide, N-(2-(pyridin-2-yl)ethyl)-3-p-menthane carboxamide, menthyl ether (e.g. (I-menthoxy)-1,2-propanediol, (I-menthoxy)-2-methyl-1,2-propanediol, 1-menthyl-methyl ether), menthyl ester (e.g. menthyl formiate, menthyl acetate, menthyl isobutyrate, menthyl lactates, L-menthyl-L-lactate, L-menthyl-D-lactate, menthyl-(2-methoxy)acetate, menthyl-(2-methoxy ethoxy)acetate, menthyl pyroglutamate), N-(4-cyano methyl phenyl)-p-menthane carboxamide, N-(2-(pyridin-2-yl)ethyl)-3-p-menthane carboxamides, menthyl carbonates (e.g. menthyl propylene glycol carbonate, menthyl ethylene glycol carbonate, menthyl glycerine carbonate or mixtures thereof), the semi-esters of mentholes with a dicarboxylic acid or their derivatives (e.g. mono-menthyl succinate, mono-menthyl glutarate, mono-menthyl malonate, O-menthyl succinic acid ester-N,N-(dimethyl)amide, O-menthyl succinic acid ester amide), menthane carboxylic acid amide (e.g. menthane carboxylic acid-N-ethylamid [WS3], N.alpha.-(menthane-carbonyl)glycine ethyl ester [WS5], menthane carboxylic acid-N-(4-cyanophenyl)amide, menthane carboxylic acid-N-(alkoxyalkyl)amide), menthone and menthone derivatives (e.g. L-menthone glycerine ketal), 2,3-dimethyl-2-(2-propyl)-butyric acid derivatives (e.g. 2,3-dimethyl-2-(2-propyl)-butyric acid-N-methyl amide [WS23]), isopulegol or its esters (1-(-)-isopulegol, 1-(-)-isopulegol acetate), menthane derivatives (e.g. p-menthane-3,8-diol), N-(4-cyano methyl phenyl)-p-menthane carboxamides, N-(2-(pyridin-2-yl)ethyl)-3-p-menthane carboxamides, cubebol or synthetic or natural mixtures containing cubebol, pyrrolidone derivates of cycloalkyl dione derivatives (e.g. 3-methyl-2(1-pyrrolidinyl)-2-cyclopentene-1-one) or tetrahydropyrimidine-2-ones (e.g. Icilin or related compounds such as those described in WO 2004/026840) or mixtures thereof.

It has been shown that using the said cooling agents in combination with a whitening or brightening agent a perceptible reduced skin stinging and improved skin feeling could be achieved.

### Liposomes

According to the invention the compositions including the whiting or brightening agent are encapsulated. The encapsulation materials are liposomes. Liposomes may be manufactured by several different methods. A common method is to dissolve a lipid or a combination of lipids in an organic solvent. Upon removal of the organic solvent and hydration, large multilamellar vesicles (MLVs) are formed. Small unilamellar vesicles (SUVs) can be produced from MLVs by techniques such as sonication, extrusion through membranes with well-defined pores, French press extrusion and homogenization. Liposomes are described in WO 02/49617 A2.

In encapsulating the whitening or brightening agent, the penetration though the skin, especially through the epidermis can be improved, thus smaller concentration of the whitening agent can be used to achieved good efficacy, and simultaneously the stinging, itching and pricking of the skin caused by the whitening agent could be reduced, because the agent is encapsulated.

### COSMETIC PREPARATIONS

The liposome compositions as defined above are useful for the manufacturing of a cosmetic preparation.

In one preferred embodiment of the invention the composition of the present invention is to be formulated into a cosmetic preparation comprises encapsulated whitening agent or brightening agent and ethanol as alcohol, wherein the whitening (or brightening) agent is preferably a skin whitening agent, preferably 4-(1-Phenylethyl) 1-,3 benzenediol. Such a composition may further comprise cis- and/or trans- 4-tert-Butylcyclohexanol (SymSitive^{®} 1609) as another preferred embodiment.

In another preferred embodiment of the invention the composition to be formulated into a cosmetic preparation comprises besides the whitening or brightening agent, a cooling agent and an alcohol, preferably the cooling agent is selected from Menthyl Lactate (Frescolat ML), Menthone Glycerin Acetal (Frescolat MGA), Menthyl Ethylamido Oxalate (Frescolat X-Cool), Menthol or mixtures thereof and the alcohol is ethanol, more preferably the cooling agent is Menthyl Lactate (Frescolat ML) and/or Menthone Glycerin Acetal (Frescolat MGA) and most preferred the cooling agent is Menthyl Lactate (Frescolat ML).

In a further preferred embodiment of the invention the composition to be formulated into a cosmetic preparation comprises besides the whitening or brightening agent, is 1, 3, 7-Trimethyl-3,7-dihydro-1H-purin-2,6-dion (Caffeine) or a derivative thereof and optionally ethanol as alcohol, preferably Caffeine and ethanol, and wherein the whitening agent is preferably 4-(1-Phenylethyl) 1-,3 benzenediol.

In another further preferred embodiment of the invention the composition to be formulated into a cosmetic preparation comprises besides the whitening or brightening agent 1,3,7-Trimethyl-3,7-dihydro-1H-purin-2,6-dion (Caffeine) and 4-tert-Butylcyclohexanol (SymSitive^{®} 1609) and wherein the whitening agent is preferably 4-(1-Phenylethyl) 1-,3 benzenediol.

Preferably the amount of the whitening or brightening agent in the cosmetic preparation is from 0.01 to 20 % b.w., preferably 0.1 to 10 % b.w., more preferably 0.5 to 2.5 % b.w., based on the total amount of the cosmetic preparation.

Preferably the amount of the cooling agent in the cosmetic preparation is from 0.05-10% b.w., preferably 0.1-5% by weight, more preferred 0.2- 2 % by weight, based on the total amount of all cooling agents.

Preferably the amount of the alcohol in the cosmetic preparation is from 2 to 10% b.w., preferably from 3 to 7% b.w., more preferably 5 to 7% b.w., based on the total amount of the cosmetic preparation.

Preferably the amount of the solubilizer in the cosmetic preparation is from 0.01 to 95 % b.w., preferably 0.02 to 50 % b.w., more preferably 0.1 to 10 % b.w., based on the total amount of the cosmetic preparation.

Preferably the amount of 1,3,7-Trimethyl-3,7-dihydro-1H-purin-2,6-dion (Caffeine) or derivatives thereof in the cosmetic preparation is from 0.1 to 10% b.w., preferably 0.3 to 7 % b.w., more preferably 0.5-5 % b.w., based on the total amount of the cosmetic preparation.

Preferably the amount of cis- and/or trans- 4-tert-Butylcyclohexanol (SymSitive^{®} 1609), in the cosmetic preparation is from 0.01 to 10% b.w., preferably 0.2 to 5 % b.w., more preferably 0.05-2 % b.w., based on the total amount of the cosmetic preparation.

In a preferred embodiment of the present invention the cosmetic preparation of the present invention is, depending on their formulation, a dermatological preparation and can be used as skin cream as day cream or night cream, milk, lotion. The preparation can also be used, depending on their formulation, in hair care compositions such as shampoos, hair coloration, hair tonics, hair lotions, hair rinses, sprays, etc.

In some instances, it is possible and advantageous to use the preparations according to the present invention as bases for pharmaceutical preparations. Preference is given, in particular, to those cosmetic or pharmaceutical and dermatological preparations in the form of a skin care, hair care or make-up product.

It has been recognized that the composition comprising the whitening or brightening agent and at least one further component selected from i) to v) as described above, can be formulated in different preparations and formulation compositions.

Thus, typical embodiments are creams, gels e.g. but not limited to hydrogels, hydro-dispersion gels, oil gels; lotions, alcoholic and aqueous/alcoholic solutions, emulsions in their various forms for example but not limited to oil in water (O/W), water in oil (W/O), mixed emulsions, PIT emulsions, Pickering emulsions, microemulsions, nano-emulsions; aerosol foams, non-aerosol foams, aerosols sprays, non-aerosol sprays, pump sprays, serums, rollons, pastes, balsams, or stick preparations.

In a further embodiment of the invention the cosmetic or pharmaceutical preparation is preferably a skin whitening preparation, a leave-on hair product, a hair coloration.

A further object of the present invention is the use of a composition as described herein with the whitening or brightening agent and at least one further component selected from i) to v) for the manufacturing of a cosmetic preparation.

As an example, the amount of the component present in such a cosmetic preparation is as follows:
a) the whitening agent is from 0.01 to 20 % b.w., preferably 0.1 to 10 % b.w., more preferably 0.5 to 2.5 % b.w;
b) the alcohol is from 2 to 10% b.w., preferably from 3 to 7% b.w., more preferably 5 to 7% b.w.;
c) the cooling agent is from 0.05- 10% b.w., preferably 0.1-5% by weight, more preferred 0.2- 2 % by weight;
d) the solubilizer is from 0.01 to 95 % b.w., preferably 0.02 to 50 % b.w., more preferably 0.1 to 10 % b.w.;
e) 1,3,7-Trimethyl-3,7-dihydro-1H-purin-2,6-dion (Caffeine) or derivatives is from 0.1 to 10% b.w., preferably 0.3 to 7 % b.w., more preferably 0.5-5 b.w.;
f) cis- and/or trans- 4-tert-Butylcyclohexanol is from 0.01 to 10% b.w., preferably 0.2 to 5 % b.w., more preferably 0.05-2 % b.w.;
in each case based on the total amount of the cosmetic preparation.

It has been further shown that when formulating the components a) to f) into cosmetic preparations stable cosmetic formulations can be obtained without disadvantage influences of the components a) to f) to the cosmetic preparations regarding the stability of the formulations or the whitening capacity of the whitening agent.

### AUXILIARY AGENTS FOR COSMETIC PREPARATIONS

The cosmetic preparations according to the invention may contain abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, ant-statics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, anticorrosives, aromas, flavouring substances, odoriferous substances, polyols, surfactants, electrolytes, organic solvents or silicone derivatives and the like as additional auxiliaries and additives.

### Surfactants

Preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C ₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcy-clohexanes.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

**Sorbitan esters.** Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

**Polyglycerol esters.** Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

**Amphoteric emulsifiers.** Other suitable emulsifiers are amphboteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents and consistency factors

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents and rheology additives

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlizing waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes and stabilizers

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### Primary sun protection filters

Primary sun protection filters in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivativesand indole derivatives.

In addition, it is advantageous to combine compounds of formula (I) with active ingredients which penetrate into the skin and protect the skin cells from inside against sunlight-induced damage and reduce the level of cutaneous matrix metalloproteases. Preferred respective ingredients, so called arylhydrocarbon receptor antagonists, are described in WO 2007/128723, incorporated herein by reference. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan^{®}HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150).

Broadband filters which are preferably combined with one or more compounds of formula (I) in a preparation according to the present invention are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine.

The compositions can comprise further typical detergent and cleansing composition ingredients such as UV-A filters filters which are preferably combined with one or more compounds of formula (I) in a preparation according to the present invention are selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds in accordance with DE 100 55 940 A1 (= WO 2002 038537 A1)

The compositions can comprise further typical detergent and cleansing composition ingredients such as UV filters which are more preferably combined with one or more compounds of formula (I) in a preparation according to the present invention are selected from the group consisting of
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCI name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds in accordance with DE 100 55 940 (= WO 02/38537).

Advantageous primary and also secondary sun protection factors are mentioned in WO 2005 123101 A1**.** Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) so that the formulation according to the invention has a light protection factor of greater than or equal to 2 (preferably greater than or equal to 5). Such formulations according to the invention are particularly suitable for protecting the skin and hair.

### Secondary sun protection filters

Besides the groups of primary sun protection filters mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodi-propionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1**.** The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### Anti-ageing actives

In the context of the invention, anti-ageing or biogenic agents are, for example antioxidants, matrix-metalloproteinase inhibitors (MMPI), skin moisturizing agents, glycosaminglycan stimulkators, anti-inflammatory agents, TRPV1 antagonists and plant extracts.

**Antioxidants.** Suitable antioxidants encompass amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.

If vitamin E and/or derivatives thereof are used as the antioxidant(s), it is advantageous to choose their concentrations from the range from about 0.001 to about 10 % b.w. based on the total weight of the formulation. If vitamin A or vitamin A derivatives or carotenes or derivatives thereof are used as the antioxidant(s), it is advantageous to choose their concentrations from the range from about 0.001 to aout 10 % b.w. based on the total weight of the formulation.

**Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02 069992 A1 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1**,** incorporated herein by reference) as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators.** Preferred compositions comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**Anti-inflammatory agents.** The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1**,** are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenan-thramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1**),** boswellic acid, phytosterols, glycyrrhizin, and licochalcone A, and/or allantoin, panthenol, lanolin, (pseudo-)ceramides [preferably Ceramide 2, hydroxypropyl bispal-mitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO2), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1**,** or indirect modulators of TRPV1 by an activation of the µ-receptor, e.g. acetyl tetrapeptide-15, are preferred.

**Desquamating agents.** The compositions may also contain desquamating agents (component b5) in amounts of about 0.1 to about 30 % b.w. preferably about 0.5 to about 15 % b.w., particularly preferably about 1 to about 10 % b.w. based on the total weight of the preparation. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosul-phonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, those described in the documents FR 2570377 A1, EP 0199636 A1, EP 0325540 A1, EP 0402072 A1, chestnut or prickly pear extracts, in particular marketed by SILAB; reducing compounds such as cysteine or cysteine precursors.

Desquamating agents which can be used are also nicotinic acid and its esters and nicotinamide, also called vitamin B3 or vitamin PP, and ascorbic acid and its precursors, as described in particular in application EP 1529522 A1**.**

**Anti-cellulite agents.** Anti-cellulite agents and lipolytic agents are preferably selected from the group consisting of those described in WO 2007/077541, and beta-adrenergic receptor agonists such as synephrine and its derivatives, and cyclohexyl carbamates described in WO 2010/097479. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents.** Formulations and products according to the present invention may also comprise one or more fat enhancing and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

### Hair growth activators or inhibitors

Formulations and products according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, formulations and products according to the present invention may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odourneutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylanc-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### Carriers and hydrotropes

Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oil and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, • - isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoff-kommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

### Preparations

Preferred compositions according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation.

### EXAMPLES

### HYDRODISPERSION GELS

Liposomes of the present invention are available through dissolving the whitening or brightening in a solubilizer. Upon hydration and/or addition to water, large multilamellar vesicles (MLVs) are formed. Small unilamellar vesicles (SUVs) can be produced from MLVs by techniques such as sonication, extrusion through membranes with well-defined pores, French press extrusion and homogenization. Liposomes of the present invention can be obtained as described in WO 02/49617 A2. The loading of such liposomes is usually in a range from about 10:90 of whitening or brightening agent to the solubilizer. In **Tables 1 and 2** liposomal formulations of the present invention are described.

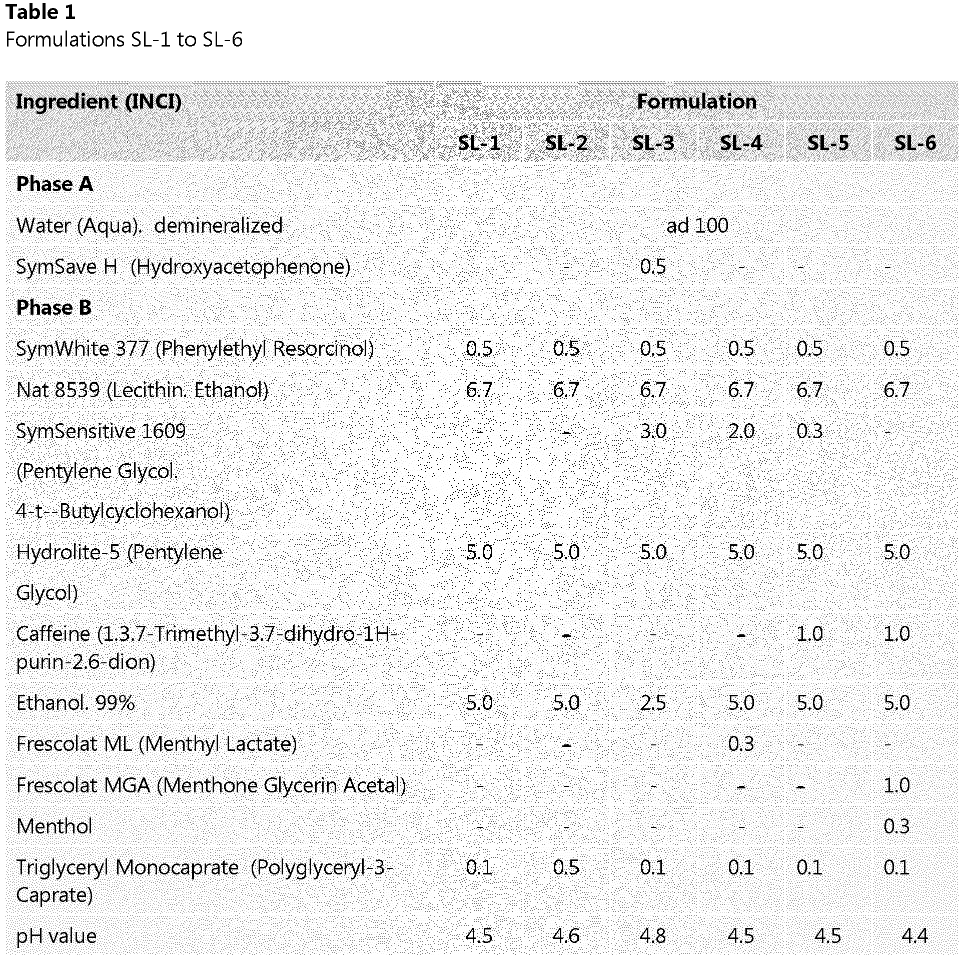

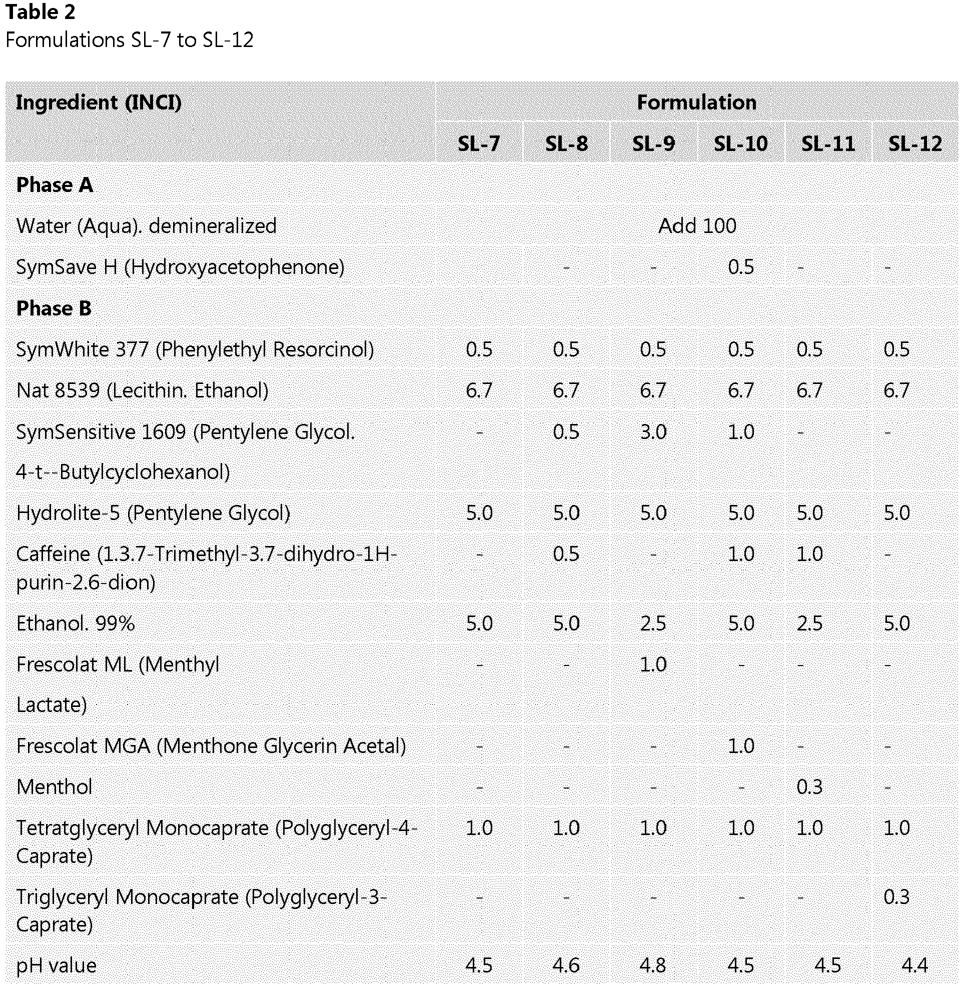

### STINGING/PICKING TEST

The stinging, picking and itching tests have been performed by a panel of 12 persons. The tested formulations were therefore applied onto the test person's skin on the left and right side of the nasolabial folds. On each side of the nasolabial folds a different formulation is spread. For that, about 2 g finger-sized portion of a formulation is circularly spread and the perception of skin stinging, picking and itching is evaluated after 5 minutes. The results are presented in the following scheme. The meaning of the symbols in the tables are as follows:

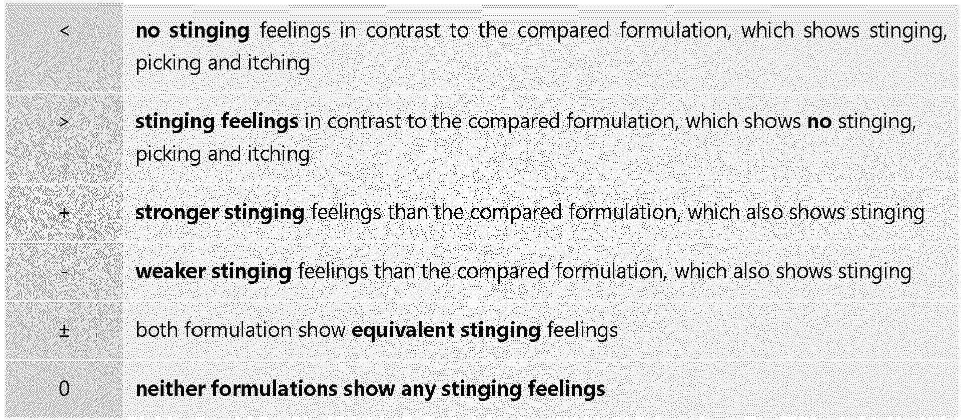

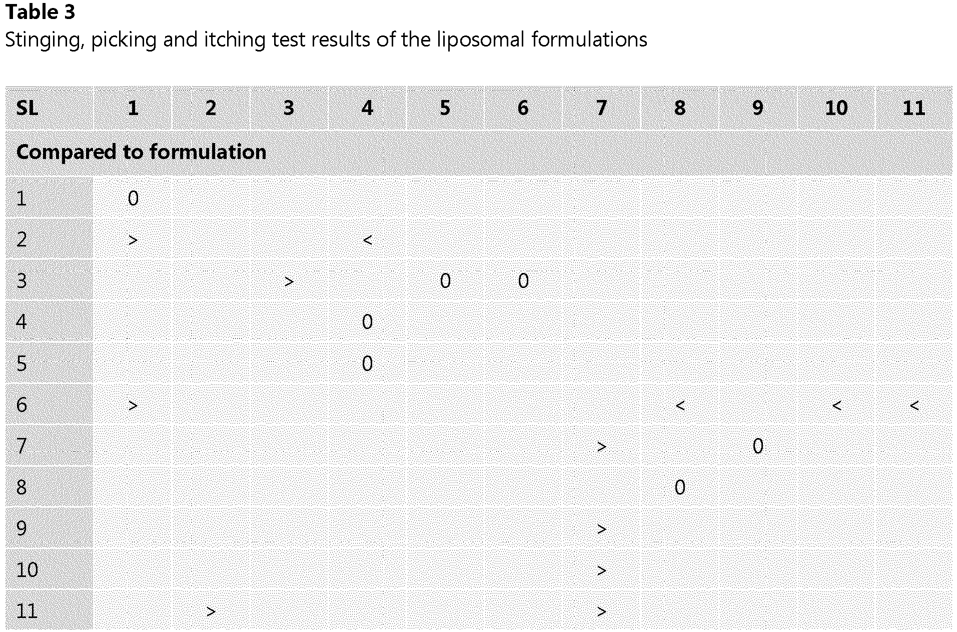

### STABILITY TEST

The formulations of the present invention have been shown to be stable at 20°C (RT) and 40°C. They have been storaged at 20°C and 40°C (in a hot cabinet) for 2, 4 and 8 weeks. A phase separation has not been occurred, but some of the formulation got cloudy and milky.

## Claims

1. Use of a liposome composition comprising
(a) at least a whiting or brightening agent, selected from the group consisting of di-phenylmethanes, sclareolide; Tetraselmis Suecica Extract; licorice extract; pomegranate extract; hinokitiol; protocatechuic acid; NAB asafetida (Ferula Foetida) extract; resveratrol oxyresveratrol resveratrol phosphate, resveratrol ferulate; ferulic acid ferulic acid phosphate; viniferol; mixtures of Saxifrage, Grape, mulberry and Scutelleria Root extracts, mixture of cucumber, apple and Scutellaria extracts or mixtures of cucumber, apple and Scutellaria, and green tea extracts; mixtures of butylene glycol/water/Denothera Biennis seed extract; evening primrose extract; fatty acid esters of ascorbic acid, ascorbyl palmitate; Euphrasia Officianalis extract, kinetin; ascorbyl glucoside; grape seed extract; tetrahydrocurcumins, Acmella Oleracea extract, Aloesin, extracts of field dock, aspergillus ferment, molasses, 4-(1-Phenylethyl) 1-,3 benzenediol, and
(b) at least one solubilizer selected from the group consisting of Tetratglyceryl Monocaprate (Polyglyceryl-4-Caprate), Triglyceryl Monocaprate (Polyglyceryl-3-Caprate), Polyglyceryl-2 Sesquiisostearate, Decyl-Glucoside Sorbitan Oleate Crosspolymer, Lauryl-Glucoside Sorbitan Oleate Crosspolymer,
and optionally
(c) at least one physiological cooling agent
for the manufacturing of a cosmetic preparation selected from the group consisting of skin whitening or brightening preparations, leave-on hair products and hair coloration products.

2. The use according to Claim 1, wherein the physiological cooling agent is selected from the group consisting of Menthyl Lactate, Menthone Glycerin Acetal, Menthyl Ethylamido Oxalate, menthol 5-methyl-2-(propane-2-yl)cyclohexyl-N-ethyloxamate, N-ethyl-p-menthane carboxamide, N-2,3-trimethyl-2-isopropyl butane amide, menthyl lactate, menthone glycerine acetal, mono-menthyl succinate, mono-menthyl glutarate, O-menthyl-glycerine, menthyl-N,N-dimethyl succinamate, N-(4-cyano methyl phenyl)-p-menthane carboxamide, N-(2-(pyridin-2-yl)ethyl)-3-p-menthane carboxamide, menthyl ether, (I-menthoxy)-2-methyl-1,2-propanediol, 1-menthyl-methyl ether), menthyl ester, menthyl formiate, menthyl acetate, menthyl isobutyrate, L-menthyl-L-lactate, L-menthyl-D-lactate, menthyl-(2-methoxy)acetate, menthyl-(2-methoxyethoxy)acetate, menthyl pyroglutamate), N-(4-cyano methyl phenyl)-p-menthane carboxamide, N-(2-(pyridin-2-yl)ethyl)-3-p-menthane carboxamides, menthyl carbonates, menthyl propylene glycol carbonate, menthyl ethylene glycol carbonate, menthyl glycerine carbonate, the semi-esters of mentholes with a dicarboxylic acid mono-menthyl succinate, mono-menthyl glutarate, mono-menthyl malonate, O-menthyl succinic acid ester-N,N-(dimethyl)amide, O-menthyl succinic acid ester amide), menthane carboxylic acid amide, menthane carboxylic acid-N-ethylamid, N-alpha-(menthane-carbonyl)glycine ethyl ester, menthane carboxylic acid-N-(4-cyanophenyl)amide, menthane carboxylic acid-N-(alkoxyalkyl)amide), menthone L-menthone glycerine ketal, 2,3-dimethyl-2-(2-propyl)-butyric acid-N-methyl amide, isopulegol or its esters (1-(-)-isopulegol, 1-(-)-isopulegol acetate), [N-(4-cyano methyl phenyl)-p-menthane carboxamides, N-(2-(pyridin-2-yl)ethyl)-3-p-menthane carboxamides, cubebol or synthetic or natural mixtures containing cubebol, 3-methyl-2(1-pyrrolidinyl)-2-cyclopentene-1-one) or tetrahydropyrimidine-2-ones or mixtures thereof.

3. The use according to Claims 1, said liposome composition further comprising
(d) ethanol,
(e) 1,3,7-Trimethyl-3,7-dihydro-1H-purin-2,6-dion (Caffeine),
(f) cis- and/or trans- 4-tert-Butylcyclohexanol,
or a mixture thereof.

4. The use according to claim 1, wherein the cosmetic preparation is a cream, gel, hydrogel, hydro-dispersion gel, oil gel; lotion, alcoholic and aqueous/alcoholic solution, (O/W) emulsion, (W/O) emulsion, mixed emulsion, PIT emulsion, Pickering emulsion, microemulsion, nano-emulsion; aerosol foam, non-aerosol foam, aerosol spray, non-aerosol spray, pump spray, serum, roll-on, paste, balsam, or stick preparation.

5. The use according to Claim 3, wherein the amount of
(a) the whitening or brightening agent is from 0.01 to 20 % b.w.,
(b) the ethanol is from 2 to 10% b.w
(c) the physiological cooling agent is from 0.05- 10% b.w.,
(d) the solubilizer is from 0.01 to 95 % b.w.,
(e) 1,3,7-Trimethyl-3,7-dihydro-1H-purin-2,6-dion (Caffeine) is from 0.1 to 10% b.w.,
(f) cis- and/or trans- 4-tert-Butylcyclohexanol is from 0.01 to 10% b.w.,
in each case based on the total amount of the cosmetic preparation.

6. The use of Claim 3, wherein the physiological cooling agent is selected from the group consisting of Menthyl Lactate, Menthone Glycerin Acetal, Menthyl Ethylamido Oxalate, menthol, 5-methyl-2-(propane-2-yl)cyclohexyl-N-ethyloxamate, N-ethyl-p-menthane carboxamide, N-2,3-trimethyl-2-isopropyl butane amide, menthyl lactate, menthone glycerine acetal, mono-menthyl succinate, mono-menthyl glutarate, O-menthyl-glycerine, menthyl-N,N-dimethyl succinamate, N-(4-cyano methyl phenyl)-p-menthane carboxamide, N-(2-(pyridin-2-yl)ethyl)-3-p-menthane carboxamide, menthyl ether, (I-menthoxy)-2-methyl-1,2-propanediol, 1-menthyl-methyl ether), menthyl ester, menthyl formiate, menthyl acetate, menthyl isobutyrate, L-menthyl-L-lactate, L-menthyl-D-lactate, menthyl-(2-methoxy)acetate, menthyl-(2-methoxy ethoxy)acetate, menthyl pyroglutamate), N-(4-cyano methyl phenyl)-p-menthane carboxamide, N-(2-(pyridin-2-yl)ethyl)-3-p-menthane carboxamides, menthyl carbonates, menthyl propylene glycol carbonate, menthyl ethylene glycol carbonate, menthyl glycerine carbonate, the semi-esters of mentholes with a dicarboxylic acid mono-menthyl succinate, mono-menthyl glutarate, mono-menthyl malonate, O-menthyl succinic acid ester-N,N-(dimethyl)amide, O-menthyl succinic acid ester amide), menthane carboxylic acid amide, menthane carboxylic acid-N-ethylamid, N-alpha-(menthane-carbonyl)glycine ethyl ester, menthane carboxylic acid-N-(4-cyanophenyl)amide, menthane carboxylic acid-N-(alkoxyalkyl)amide), menthone, L-menthone glycerine ketal), 2,3-dimethyl-2-(2-propyl)-butyric acid-N-methyl amide, isopulegol or its esters (1-(-)-isopulegol, 1-(-)-isopulegol acetate), N-(4-cyano methyl phenyl)-p-menthane carboxamides, N-(2-(pyridin-2-yl)ethyl)-3-p-menthane carboxamides, cubebol or synthetic or natural mixtures containing cubebol, 3-methyl-2(1-pyrrolidinyl)-2-cyclopentene-1-one) or tetrahydropyrimidine-2-ones or mixtures thereof.

## Patentansprüche

1. Verwendung einer Liposomenzusammensetzung, umfassend
(a) mindestens ein weißmachendes oder aufhellendes Mittel, ausgewählt aus der Gruppe bestehend aus Diphenylmethanen, Sclareolid; Tetraselmis Suecica Extrakt; Süßholzextrakt; Granatapfelextrakt; Hinokitiol; Protocatechusäure; NAB Asafetida (Ferula Foetida) Extrakt; Resveratrol-Oxyresveratrol, Resveratrol-Phosphat, Resveratrol-Ferulat; Ferulasäure-Ferulasäure-Phosphat; Viniferol; Mischungen aus Steinbrech-, Trauben-, Maulbeer- und Scutelleria-Wurzel-Extrakten, Mischungen aus Gurken-, Apfel- und Scutellaria-Extrakten oder Mischungen aus Gurken-, Apfel- und Scutellaria- und Grüntee-Extrakten; Gemische aus Butylenglykol/Wasser/Denothera-Biennis-Samenextrakt; Nachtkerzenextrakt; Fettsäureester der Ascorbinsäure, Ascorbylpalmitat; Euphrasia-Officianalis-Extrakt, Kinetin; Ascorbylglucosid; Traubenkernextrakt; Tetrahydrocurcumine, Acmella-Oleracea-Extrakt, Aloesin, Extrakte von Ackerkratzdorn, Aspergillus-Ferment, Melasse, 4-(1-Phenylethyl)-1,3-Benzoldiol, und
(b) mindestens einen Lösungsvermittler, ausgewählt aus der Gruppe bestehend aus Tetratglycerylmonocaprat (Polyglyceryl-4-Caprat), Triglycerylmonocaprat (Polyglyceryl-3-Caprat), Polyglyceryl-2-Sesquiisostearat, Decyl-Glucosid-Sorbitan Oleat-Kreuzpolymer, Lauryl-Glucosid-Sorbitan Oleat-Kreuzpolymer, und gegebenenfalls
(c) mindestens ein physiologisches Kühlmittel
zur Herstellung eines kosmetischen Präparats, ausgewählt aus der Gruppe, die aus hautaufhellenden oder aufhellenden Präparaten, Leave-on-Haarprodukten und Haarfärbeprodukten besteht.

2. Verwendung nach Anspruch 1, wobei das physiologische Kühlmittel ausgewählt ist aus der Gruppe bestehend aus Menthyllactat, Menthonglycerinacetal, Menthylethylamidooxalat, Menthol-5-methyl-2-(propan-2-yl)cyclohexyl-N-ethyloxamat, N-Ethyl-p-Menthancarboxamid, N-2,3-Trimethyl-2-isopropylbutanamid, Menthyllactat, Menthonglycerinacetal, Mono-Menthylsuccinat, Mono-Menthylglutarat, O-Menthylglycerin, Menthyl-N,N-dimethylsuccinamat, N-(4-Cyanmethylphenyl)-p-menthan-carboxamid, N-(2-(Pyridin-2-yl)ethyl)-3-p-menthan-carboxamid, Menthylether, (I-Menthoxy)-2-methyl-1,2-propandiol, 1-Menthyl-methylether), Menthylester, Menthylformiat, Menthylacetat, Menthylisobutyrat, L-Menthyl-L-lactat, L-Menthyl-D-lactat, Menthyl-(2-methoxy)acetat, Menthyl-(2-methoxyethoxy)acetat, Menthylpyroglutamat), N-(4-Cyanmethylphenyl)-p-menthancarboxamid, N-(2-(Pyridin-2-yl)ethyl)-3-p-menthancarboxamide, Menthylcarbonate, Menthylpropylenglykolcarbonat, Menthylethylenglykolcarbonat, Menthylglycerincarbonat, Halbester von Mentholen mit einer Dicarbonsäure, Monomenthylsuccinat, Monomenthylglutarat, Monomenthylmalonat, O-Menthylbernsteinsäureester-N,N-(dimethyl)amid, O-Menthylbernsteinsäureesteramid), Menthancarbonsäureamid, Menthancarbonsäure-N-ethylamid, N-alpha-(Menthancarbonyl)glycinethylester, Menthancarbonsäure-N-(4-cyanophenyl)amid, Menthancarbonsäure-N-(alkoxyalkyl)amid), Menthonglyceringetal, 2,3-Dimethyl-2-(2-propyl)-buttersäure-N-methylamid, Isopulegol oder seine Ester (1-(-)-Isopulegol, 1-(-)-Isopulegolacetat), [N-(4-Cyanmethylphenyl)-p-menthancarboxamide, N-(2-(Pyridin-2-yl)ethyl)-3-p-menthan-carboxamide, Cubebol oder synthetische oder natürliche Mischungen, die Cubebol, 3-Methyl-2(1-pyrrolidinyl)-2-cyclopenten-1-on enthalten) oder Tetrahydropyrimidin-2-one oder Mischungen davon.

3. Verwendung nach Anspruch 1, wobei die Liposomenzusammensetzung ferner umfasst
(d) Ethanol,
(e) 1,3,7-Trimethyl-3,7-dihydro-1H-purin-2,6-dion (Koffein),
(f) cis- und/oder trans- 4-tert-Butylcyclohexanol,
oder ein Gemisch davon.

4. Verwendung nach Anspruch 1, wobei die kosmetische Zubereitung eine Creme, ein Gel, ein Hydrogel, ein Hydrodispersionsgel, ein Ölgel, eine Lotion, eine alkoholische und eine wässrig/alkoholische Lösung, eine (O/W)-Emulsion, eine (W/O)-Emulsion, eine gemischte Emulsion, eine PIT-Emulsion, eine Pickering-Emulsion, eine Mikroemulsion, eine Nanoemulsion, ein Aerosolschaum, ein Nicht-Aerosolschaum, ein Aerosolspray, ein Nicht-Aerosolspray, ein Pumpspray, ein Serum, ein Roll-on, eine Paste, ein Balsam oder eine Stickzubereitung ist.

5. Verwendung nach Anspruch 3, wobei die Menge an
(a) Bleich- oder Aufhellungsmittels von 0,01 bis 20 Gew.-%,
(b) Ethanol von 2 bis 10 Gew.-%,
(c) physiologischen Kühlmittels von 0,05 bis 10 Gew.-%,
(d) Lösungsvermittler von 0,01 bis 95 Gew.-%,
(e) 1,3,7-Trimethyl-3,7-dihydro-1H-purin-2,6-dion (Koffein) von 0,1 bis 10 Gew.-%, sowie
(f) cis- und/oder trans-4-tert-Butylcyclohexanol von 0,01 bis 10 Gew.-%, beträgt - jeweils bezogen auf die Gesamtmenge der kosmetischen Zubereitung.

6. Verwendung nach Anspruch 3, **dadurch gekennzeichnet, dass** das physiologische Kühlmittel ausgewählt ist aus der Gruppe bestehend aus Menthyl Lactate, Menthone Glycerin Acetal, Menthyl Ethylamido Oxalate, Menthol, 5-Methyl-2-(propan-2-yl)cyclohexyl-N-ethyloxamate, N-Ethyl-p-Menthancarboxamid, N-2,3-Trimethyl-2-isopropylbutanamid, Menthyllactat, Menthonglycerinacetal, Mono-Menthylsuccinat, Mono-Menthylglutarat, O-Menthylglycerin, Menthyl-N,N-dimethylsuccinamat, N-(4-Cyanmethylphenyl)-p-menthan-carboxamid, N-(2-(Pyridin-2-yl)ethyl)-3-p-menthan-carboxamid, Menthylether, (I-Menthoxy)-2-methyl-1,2-propandiol, 1-Menthyl-methylether), Menthylester, Menthylformiat, Menthylacetat, Menthylisobutyrat, L-Menthyl-L-lactat, L-Menthyl-D-lactat, Menthyl-(2-methoxy)acetat, Menthyl-(2-methoxyethoxy)acetat, Menthylpyroglutamat), N-(4-Cyanmethylphenyl)-p-menthancarboxamid, N-(2-(Pyridin-2-yl)ethyl)-3-p-menthancarboxamide, Menthylcarbonate, Menthylpropylenglykolcarbonat, Menthylethylenglykolcarbonat, Menthylglycercarbonat, Halbester von Mentholen mit einer Dicarbonsäure, Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamid, Menthancarbonsäure-N-ethylamid, N-alpha-(Menthancarbonyl)glycinethylester, Menthancarbonsäure-N-(4-cyanophenyl)amid, Menthancarbonsäure-N-(alkoxyalkyl)amid), Menthon, L-Menthonglycerinketal), 2,3-Dimethyl-2-(2-propyl)-buttersäure-N-methylamid, Isopulegol oder seine Ester (1-(-)-Isopulegol, 1-(-)-Isopulegolacetat), N-(4-Cyanmethylphenyl)-p-Menthancarboxamide, N-(2-(Pyridin-2-yl)ethyl)-3-p-menthan-carboxamide, Cubebol oder synthetische oder natürliche Mischungen, die Cubebol, 3-Methyl-2(1-pyrrolidinyl)-2-cyclopenten-1-on) oder Tetrahydropyrimidin-2-one oder Mischungen davon enthalten.

## Revendications

1. Utilisation d'une composition de liposomes comprenant
(a) au moins un agent blanchissant ou éclaircissant, choisi dans le groupe constitué par les diphénylméthanes, le sclaréolide ; l'extrait de Tetraselmis Suecica ; l'extrait de réglisse ; l'extrait de grenade ; l'hinokitiol ; l'acide protocatéchuique ; l'extrait d'asafetida NAB (Ferula Foetida) ; oxyresvératrol ; phosphate de resvératrol, férulate de resvératrol ; acide férulique ; phosphate d'acide férulique ; viniférol ; mélanges d'extraits de racines de saxifrage, de raisin, de mûrier et de Scutelleria, mélange d'extraits de concombre, de pomme et de Scutellaria ou mélanges d'extraits de concombre, de pomme et de Scutellaria, et de thé vert ; mélanges de butylène glycol/eau/extrait de graines de Denothera Biennis ; extrait d'onagre ; esters d'acides gras de l'acide ascorbique, palmitate d'ascorbyle ; extrait d'Euphrasia Officianalis, kinétine ; glucoside d'ascorbyle ; extrait de pépins de raisin ; tétrahydro-curcumines, extrait d'Acmella Oleracea, aloesine, extraits de rumex, ferment d'aspergillus, mélasse, 4-(1-Phenylethyl) 1-,3 benzenediol, et
(b) au moins un agent solubilisant choisi dans le groupe constitué par le monocaprate de tétratglycéryle (polyglycéryl-4-caprate), le monocaprate de triglycéryle (polyglycéryl-3-caprate), le sesquiisostéarate de polyglycéryle-2, le polymère croisé d'oléate de décylglucoside et de sorbitane, le polymère croisé d'oléate de laurylglucoside et de sorbitane, et éventuellement
(c) au moins un agent de refroidissement physiologique
pour la fabrication d'une préparation cosmétique choisie dans le groupe constitué par les préparations de blanchiment ou d'éclaircissement de la peau, les produits capillaires sans rinçage et les produits de coloration des cheveux.

2. Utilisation selon la revendication 1, dans laquelle l'agent de refroidissement physiologique est choisi dans le groupe constitué par le lactate de menthe, l'acétal de glycérine de menthone, l'éthylamido oxalate de menthe, le 5-méthyl-2-(propane-2-yl)cyclohexyl-N-éthyloxamate de menthol, N-éthyl-p-menthane carboxamide, N-2,3-triméthyl-2-isopropyl butane amide, lactate de menthyle, acétal de glycérine de menthyle, succinate de mono-menthyle, glutarate de mono-menthyle, O-menthyl-glycérine, N,N-diméthyl succinamate de menthyle, N-(4-cyano méthyl phényl)-p-menthane carboxamide, N-(2-(pyridin-2-yl)éthyl)-3-p-menthane carboxamide, éther de menthyle, (I-menthoxy)-2-méthyl-1,2-propanediol, 1-menthyl-méthyl éther), ester de menthyle, formiate de menthyle, acétate de menthyle, isobutyrate de menthyle, L-menthyl-L-lactate, L-menthyl-D-lactate, menthyl-(2-méthoxy)acétate, menthyl-(2-méthoxyéthoxy)acétate, pyroglutamate de menthyle), N-(4-cyano méthyl phényl)-p-menthane carboxamide, N-(2-(pyridin-2-yl)éthyl)-3-p-menthane carboxamides, carbonates de menthyle, carbonate de propylène glycol de menthyle, carbonate d'éthylène glycol de menthyle, carbonate de glycérine de menthyle, les semi-esters de mentholes avec un acide dicarboxylique : succinate de mono-menthyle, glutarate de mono-menthyle, malonate de mono-menthyle, l'ester d'acide O-menthyl succinique-N,N-(diméthyl)amide, l'ester d'acide O-menthyl succinique amide), l'amide d'acide menthane carboxylique, l'acide menthane carboxylique-N-éthylamide, l'ester éthylique de N-alpha-(menthane-carbonyl)glycine, l'acide menthane carboxylique-N-(4-cyanophényl)amide, menthane carboxylique-N-(alcoxyalkyl)amide), menthone L-menthone glycérine cétal, acide 2,3-diméthyl-2-(2-propyl)-butyrique-N-méthyl amide, isopulégol ou ses esters (1-(-)-isopulégol, acétate de 1-(-)-isopulégol), [N-(4-cyano méthyl phényl)-p-menthane carboxamides, N-(2-(pyridin-2-yl)éthyl)-3-p-menthane carboxamides, cubébol ou mélanges synthétiques ou naturels contenant du cubébol, 3-méthyl-2(1-pyrrolidinyl)-2-cyclopentène-1-one) ou tétrahydropyrimidine-2-ones ou leurs mélanges.

3. Utilisation selon la revendication 1, ladite composition de liposomes comprenant en outre
(d) de l'éthanol,
(e) du 1,3,7-Triméthyl-3,7-dihydro-1H-purine-2,6-dion (Caféine),
(f) du cis- et/ou trans- 4-tert-butylcyclohexanol,
ou un mélange de ceux-ci.

4. Utilisation selon la revendication 1, dans laquelle la préparation cosmétique est une crème, un gel, un hydrogel, un gel hydrodispersé, un gel huileux ; une lotion, une solution alcoolique et aqueuse/alcoolique, une émulsion (H/E), une émulsion (E/H), une émulsion mixte, une émulsion PIT, une émulsion Pickering, une microémulsion, une nano-émulsion ; une mousse aérosol, une mousse non aérosol, un spray aérosol, un spray non aérosol, un spray à pompe, un sérum, un roll-on, une pâte, un baume ou une préparation en stick.

5. Utilisation selon la revendication 3, dans laquelle la quantité de
(a) l'agent de blanchiment ou d'éclaircissement est de 0,01 à 20 % en poids,
(b) l'éthanol est de 2 à 10% en poids
(c) l'agent de refroidissement physiologique est de 0,05 à 10 % en poids,
(d) le solubilisant est de 0,01 à 95 % en poids,
(e) le 1,3,7-Triméthyl-3,7-dihydro-1H-purine-2,6-dion (Caféine) est de 0,1 à 10% en poids, et
(f) le cis- et/ou trans- 4-tert-Butylcyclohexanol est de 0,01 à 10% en poids, dans chaque cas par rapport à la quantité totale de la préparation cosmétique.

6. Utilisation de la revendication 3, dans laquelle l'agent de refroidissement physiologique est choisi dans le groupe constitué par le lactate de menthyle, l'acétal de glycérine de menthone, l'éthylamido oxalate de menthyle, le menthol, le 5-méthyl-2-(propane-2-yl)cyclohexyl-N-éthyloxamate, N-éthyl-p-menthane carboxamide, N-2,3-triméthyl-2-isopropyl butane amide, lactate de menthyle, acétal de glycérine de menthyle, succinate de mono-menthyle, glutarate de mono-menthyle, O-menthyl-glycérine, N,N-diméthyl succinamate de menthyle, N-(4-cyano méthyl phényl)-p-menthane carboxamide, N-(2-(pyridin-2-yl)éthyl)-3-p-menthane carboxamide, éther de menthyle, (I-menthoxy)-2-méthyl-1,2-propanediol, 1-menthyl-méthyl éther), ester de menthyle, formiate de menthyle, acétate de menthyle, isobutyrate de menthyle, L-lactate de L-menthyle, D-lactate de L-menthyle, acétate de menthyle-(2-méthoxy), acétate de menthyle-(2-méthoxy éthoxy), pyroglutamate de menthyle), N-(4-cyano méthyl phényl)-p-menthane carboxamide, N-(2-(pyridin-2-yl)éthyl)-3-p-menthane carboxamides, carbonates de menthyle, carbonate de propylène glycol de menthyle, carbonate d'éthylène glycol de menthyle, carbonate de glycérine de menthyle, les semi-esters de mentholes avec un acide dicarboxylique : succinate de mono-menthyle, glutarate de mono-menthyle, malonate de mono-menthyle, l'ester d'acide O-menthyl succinique-N,N-(diméthyl)amide, l'ester d'acide O-menthyl succinique amide), l'amide d'acide menthane carboxylique, l'acide menthane carboxylique-N-éthylamide, l'ester éthylique de N-alpha-(menthane-carbonyl)glycine, l'acide menthane carboxylique-N-(4-cyanophényl)amide, menthone, cétal de glycérine de L-menthone), N-méthylamide de l'acide 2,3-diméthyl-2-(2-propyl)-butyrique, isopulégol ou ses esters (1-(-)-isopulégol, acétate de 1-(-)-isopulégol), N-(4-cyano méthyl phényl)-p-menthane carboxamides, N-(2-(pyridin-2-yl)éthyl)-3-p-menthane carboxamides, cubébol ou mélanges synthétiques ou naturels contenant du cubébol, 3-méthyl-2(1-pyrrolidinyl)-2-cyclopentène-1-one) ou tétrahydropyrimidine-2-o
